(19) **Europäisches Patentamt / European Patent Office / Office européen des brevets**

(11) **EP 2 667 222 B1**

(12) # EUROPEAN PATENT SPECIFICATION

(45) Date of publication and mention
of the grant of the patent:
**21.03.2018 Bulletin 2018/12**

(51) Int Cl.:
***G01T 1/161*** *(2006.01)*

(21) Application number: **12736651.6**

(22) Date of filing: **20.01.2012**

(86) International application number:
**PCT/JP2012/051243**

(87) International publication number:
**WO 2012/099248 (26.07.2012 Gazette 2012/30)**

(54) **POSITRON EMISSION COMPUTED TOMOGRAPHY DEVICE, METHOD EXECUTED BY POSITRON EMISSION COMPUTED TOMOGRAPHY DEVICE, AND PROGRAM**

TOMOGRAPHIEVORRICHTUNG MIT POSITRONENEMISSIONSBERECHNUNG, VON DER TOMOGRAPHIEVORRICHTUNG MIT POSITRONENEMISSIONSBERECHNUNG AUSGEFÜHRTES VERFAHREN UND PROGRAMM DAFÜR

DISPOSITIF DE TOMODENSITOMÉTRIE PAR ÉMISSION DE POSITONS, PROCÉDÉ EXÉCUTÉ PAR UN DISPOSITIF DE TOMODENSITOMÉTRIE PAR ÉMISSION DE POSITONS, ET PROGRAMME

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.01.2011 US 201113010237**

(43) Date of publication of application:
**27.11.2013 Bulletin 2013/48**

(73) Proprietor: **Toshiba Medical Systems Corporation Otawara-shi, Tochigi-ken 324-8550 (JP)**

(72) Inventor: **GAGNON, Daniel
Vernon Hills
Illinois 60061 (US)**

(74) Representative: **Moreland, David et al
Marks & Clerk LLP
Aurora
120 Bothwell Street
Glasgow G2 7JS (GB)**

(56) References cited:
**JP-A- 3 251 784      JP-A- 4 170 941
JP-A- 5 130 984      JP-A- 5 130 984**

JP-A- 8 313 636      JP-A- 2001 194 459

- ENGELKEN F J ET AL: "Determining Optimal Acquisition Parameters for Computed Tomography Coronary Angiography: Evaluation of a Software-assisted, Breathhold Exam Simulation", ACADEMIC RADIOLOGY, vol. 16, no. 2, February 2009 (2009-02), pages 239-243, XP002729627, ELSEVIER SCIENCE B.V. NETHERLANDS ISSN: 1076-6332
- FLISCH A ET AL: "Efficient Volume Digitizing with Adaptive Computerized Tomography", NDT.net INTERNET CITATION, May 2004 (2004-05), page 4PP, XP002414778, Retrieved from the Internet: URL:http://www.ndt.net/article/ct2003/v17/ v17.htm [retrieved on 2007-01-15]
- KORTESNIEMI M ET AL: "Radiation exposure in body computed tomography examinations of trauma patients", PHYSICS IN MEDICINE AND BIOLOGY, vol. 51, no. 12, 21 June 2006 (2006-06-21) , pages 3269-3282, XP020095810, INSTITUTE OF PHYSICS PUBLISHING, BRISTOL GB ISSN: 0031-9155, DOI: 10.1088/0031-9155/51/12/018
- G. BRIX ET AL: "Radiation exposure in multi-slice versus single-slice spiral CT: results of a nationwide survey", EUROPEAN RADIOLOGY, vol. 13, no. 8, 1 August 2003 (2003-08-01) , pages 1979-1991, XP055139707, ISSN: 0938-7994, DOI: 10.1007/s00330-003-1883-y

## Description

Field

Embodiments described herein relate to a positron emission computed tomography apparatus, a method performed by a positron emission computed tomography apparatus, and a program.

Background

The use of gamma ray detectors, and positron emission tomography or PET (Positron Emission Tomography) detectors in particular, is growing in the field of medical imaging. In PET imaging, a radiopharmaceutical agent is introduced into a subject to be imaged via injection, inhalation, or ingestion. After administration of the radiopharmaceutical agent, the physical and bio-molecular properties of the agent cause it to concentrate at specific locations in the human body. The actual spatial distribution of the agent, the intensity of the region of accumulation of the agent, and the kinetics of the process from administration to its eventual elimination are all factors that may have clinical significance. During this process, a positron emitter attached to the radiopharmaceutical agent will emit positrons according to the physical properties of the isotope, such as half-life, branching ratio, etc.

The radionuclide emits positrons, and when an emitted positron collides with an electron, an annihilation event occurs, wherein the positron and the electron are destroyed. Most of the time, an annihilation event produces two gamma rays (at 511 keV) emitted at substantially 180 degrees apart.

By detecting the two gamma rays, and drawing a line between the locations where they are detected, i.e., the line-of-response (LOR), the likely location of the original annihilation can be derived. While this process will only identify a line of possible interaction, by accumulating a large number of such lines and performing a tomography reconstruction process, the original distribution can be estimated. In addition to the locations of the two scintillation events, if accurate timing (within few hundred picoseconds) is available, a TOF (time-of-flight) calculation can add more information regarding the likely position of the annihilation event along the LOR. Limitations in the timing resolution of the PET device will determine the accuracy of the positioning along the LOR. Limitations in the determination of the locations of the original scintillation events will determine the ultimate spatial resolution of the PET device, while the specific characteristics of the isotope (e.g., energy of the positron) will also contribute (via positron range and co-linearity of the two gamma rays) to the determination of the spatial resolution of the specific agent.

Citation List

The Institution of Electrical Engineers, Stevenage, GB; February 2009 (2009-02), Engelken F J et al: "Determining Optimal Acquisition Parameters for Computed Tomography Coronary Angiograph: Evaluation of a Software-assisted, Breathhold Exam Simulation", Database accession no. 11779807; & Academic Radiology Elsevier Science B.V. Netherlands, vol. 16, no. 2, pages 239 to 243, ISSN: 1076-6332, DOI: 10.1016/J.ACRA.2008.08.016 discloses determining the optimal scanning parameters (gantry rotation time and pitch) depending on the minimum and maximum heart rate during the scan simulation. To find a given heart rate, the system used the highest and lowest value in the range of heart rates during the simulation and added a safety margin. On the basis of the heart rate variance and the expected scan time, it determined how many segments to use, the software calculated for each possible gantry rotation time the average temporal acquisition window width over the observed heart rate range. The gantry rotation time with the lowest temporal acquisition window width was then chosen. Pitch was adjusted to minimize patient exposure while allowing a sufficient number of heartbeats to be covered. These settings were defined as optimal for the analysis.

The invention is in the apparatus of Claim 1, the method of Claim 2 and the program of Claim 3.

Brief Description of Drawings

FIG. 1 is a diagram illustrating the sensitivity of a PET device for different events;
FIG. 2 is a diagram illustrating an axial sensitivity profile of a typical PET scanner over the length of one FOV in the axial direction;
FIG. 3 is a diagram illustrating the axial sensitivity profile for a multi-step PET scan having 50% overlap for each scanning step;
FIG. 4 is a diagram illustrating overshoot of the targeted length to be scanned using a fixed step-size;
FIG. 5 is a diagram illustrating overshoot of the targeted length to be scanned using a fixed step-size;
FIG. 6 is a diagram illustrating a scanning method according to a first embodiment;

FIG. 7 is a diagram illustrating a scanning method according to a second embodiment in which undershoot and overshoot are eliminated by adjusting the overlap percentage;
FIG. 8 is a diagram illustrating the initial sensitivity profile and the nominal sensitivity profile;
FIG. 9A is a flowchart illustrating the steps in the first embodiment;
FIG. 9B is a flowchart illustrating the steps in the second embodiment;
FIG. 9C is a flowchart illustrating the steps in a third embodiment; and
FIG. 10 is a schematic drawing of a gamma ray detection system that can be used to obtain a gamma ray or PET event information according to the embodiments. Description of Embodiments

A method that is performed by a PET device according to an embodiment, a PET apparatus, and a program will be described with reference to the drawings. A positron emission computed tomography apparatus according to an embodiment includes a minimum number deriving unit, an excess length deriving unit, and a starting point deriving unit. The minimum number deriving unit derives a minimum number that is the number of scans in a case where multiple scans are successively performed and that is necessary to image at least an axial extent of a region of interest. The excess length deriving unit derives an excess length that is excessively scanned by the scans in the axial direction based on the minimum number of scans, the axial extent of the region of interest, and the length of an axial FOV. The starting point deriving unit derives a starting point of a first scan of the scans so that the excess length is equally allocated on each end of the region of interest in the axial direction.

A gamma ray detection process performed by a PET device (hereinafter, PET scanner) must be repeated for a large number of annihilation events. While each imaging case must be analyzed to determine how many counts (i.e., paired events) are required to support the imaging task, current practice dictates that a typical 100-cm long, 18FDG (fluoro-deoxyglucose) study needs to accumulate several hundred million counts. The time required to accumulate this number of counts is determined by the injected dose of the agent and the sensitivity and counting capacity of the PET device.

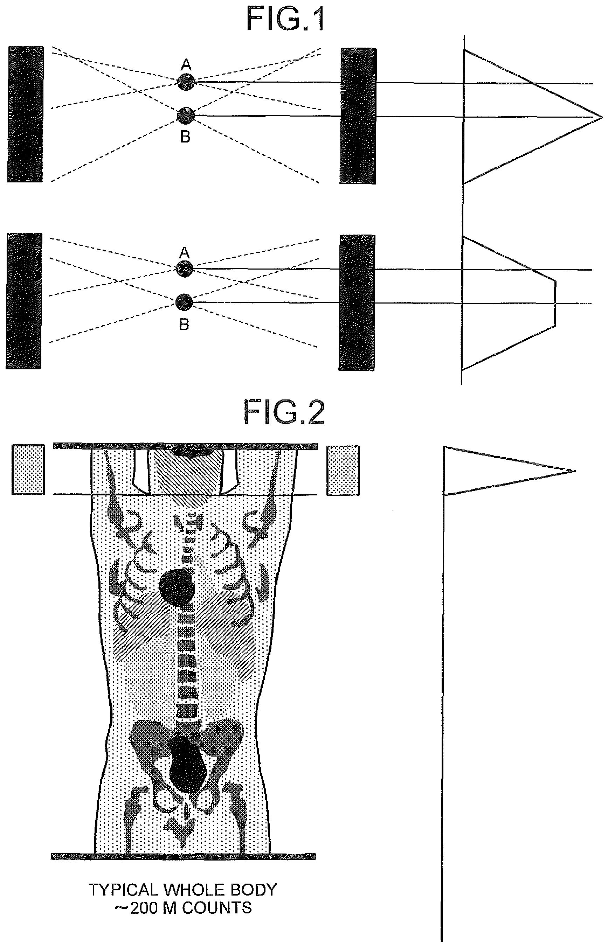

The PET scanner will acquire counts in a three-dimensional mode. This means that each LOR emitted from a subject and crossing two detector elements is potentially detected. Thus, for example, the two emission points A and B disposed as indicated in FIG. 1 have different probabilities of being detected. The point B in the middle of the center of the axial line has the maximum probability of being detected as it has the largest solid angle, including the two legs of the annihilation event. The point A supports a smaller angle, is a point right at the edge of the axial FOV, and has a minimal probability of being detected. This analysis is used to determine the overall scanner sensitivity. The sensitivity has a triangular shape typical of a completely open 3D scanner, as shown on the right side of FIG. 1.

A special case of this situation is when, by electronics design or otherwise, the largest angles are cut off from the analysis since large oblique angles of incidence may bring some challenges for some reconstruction algorithms. In this case, the typical triangle would be flattened at some point related to the cut-off angle.

This triangular axial sensitivity profile is useful as the typical 100 cm PET studies require more than one axial FOV of the PET scanner. Several bed positions need to be added. The PET scanner FOV with respect to the total length requested by the user is shown. Usually, 50% overlap between steps is optimal as it creates flat sensitivity over the central portion of the image, as show in FIG. 2.

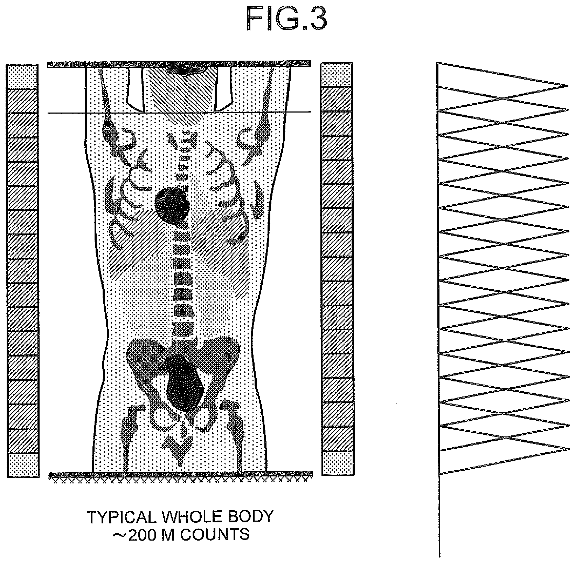

A repeat of the same FOV with a 50% overlap per scan is illustrated in FIG. 3.

A fixed step size will likely create an undershoot or an overshoot of the targeted length. Since under-sampling the region of interest is not an option, the PET scanner is likely to take an extra step to cover the entire area. In this case, the overshoot area, shown at the bottom of FIG. 3, is small. In practice, the imaging system may only obtain achievable length by forcing the user to step through the possible discrete length. Either way, the total length of the PET image will slightly overshoot the length the clinician would have selected.

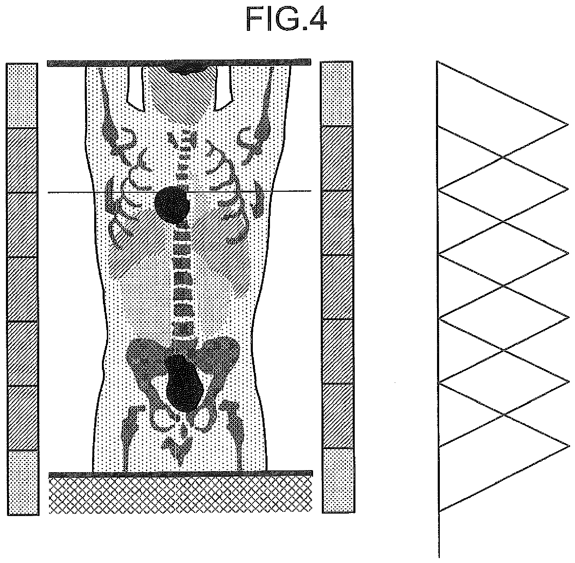

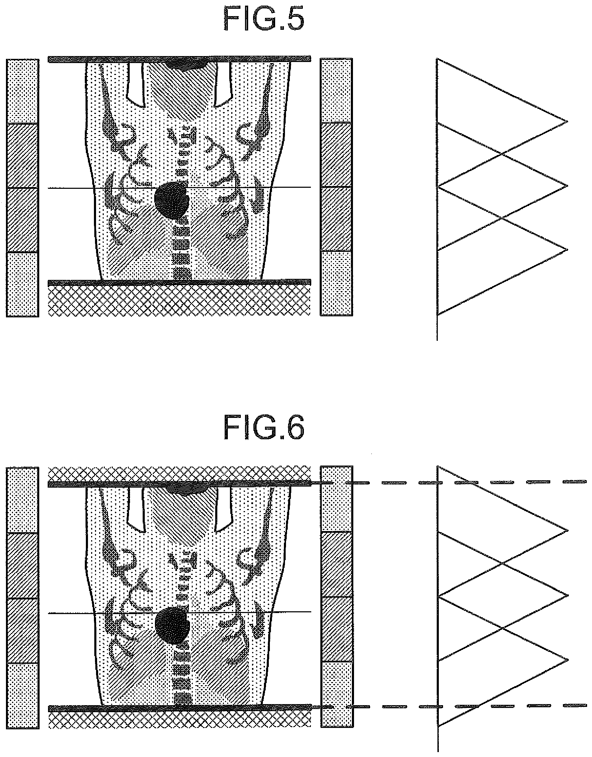

This effect will be exacerbated in PET scanners with a larger axial FOV, as shown in FIG. 4, in which the overshoot area is visibly more important. This effect is also proportionally increased when the requested area of interest is shorter, as shown in FIG. 5.

An alternative approach is to design a system with a continuous bed motion in which the speed profile and the total length scanned can be controlled more precisely. However, continuous bed motion is not always available in all systems and definitely adds complexity to data acquisition and reconstruction.

Thus, as illustrated in FIGS. 3-5, in a step-and-shoot system, a significant amount of time and counts can be wasted outside of the area of interest. This means that the same imaging resources could have been used more efficiently.

Conventional PET scanners have an axial FOV between 16 and 22 cm to cover a typical 100 cm length. With an overlap of 50%, 8 to 12 steps are typically required, with possibly 8-12% of imaging resources being wasted outside the area of interest. In other studies, like lung or head-neck imaging, the region of interest is generally 30 to 50 cm, where the waste of imaging resources can be as high as 20-30%.

Embodiments described herein relate to a new method of optimizing the step-size in multi-step PET imaging using a PET scanner.

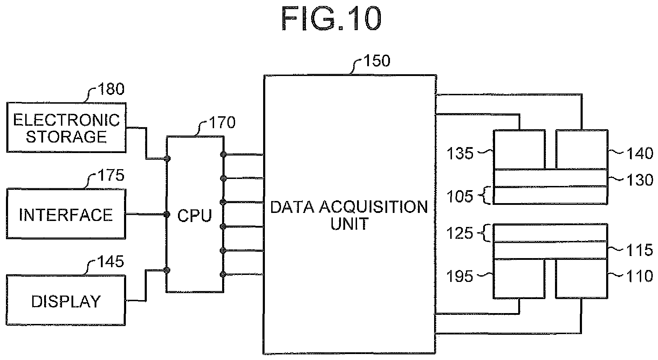

As described below, a PET scanner according to a first embodiment includes a minimum number deriving unit, an excess length deriving unit, and a starting point deriving unit. The minimum number deriving unit derives a minimum number that is the number of scans in a case where multiple scans are successively performed while each scan overlaps an adjacent scan by a predetermined percentage and that is necessary to image at least an axial extent of a region of interest. The excess length deriving unit derives an excess length that is excessively scanned by the scans in the axial direction based on the minimum number of scans, the axial extent of the region of interest, and the length of the axial FOV. The starting point deriving unit derives a starting point of a first scan of the scans so that the excess length is equally allocated on each end of the region of interest in the axial direction. The PET scanner according to the first embodiment further includes a scanning unit configured to scan a subject by starting the minimum number of scans from the starting point. Each of these units is included in a CPU (Central Processing Unit) 170 described below using FIG. 10 and coordinates with a detector module etc.

A method of using a PET scanner of the first embodiment, for example, by imaging a region of interest in a subject, which is a region of interest having an axial extent greater than the length of an axial FOV of a PET scanner, includes: (1) a step of determining a minimum number that is the number of scans of overlapping scans (multiple scans are successively performed while each scan overlaps an adjacent scan by a predetermined percentage) and that is necessary to image at least an axial extent of a region of interest; (2) a step of determining an excess length (total amount) that is excessively scanned by the scans in the axial direction based on the minimum number of scans determined in step (1), the axial extent of the region of interest, and the length of the axial FOV; and (3) a step of determining an initial starting point of a first scan of the scans successively performed so that the excess length (total amount) is equally allocated on each end of the subject in the axial direction. Each of the scans successively performed has the same axial length equal to the length of the axial FOV of the PET scanner. Each scan partly overlaps an adjacent scan by a predetermined percentage of the axial length of each scan.

Furthermore, as described below, the PET scanner according to an example includes a combination deriving unit. When multiple scans are successively performed while each scan overlaps an adjacent scan and a total length of the scans exactly equals the axial extent of a region of interest, the combination deriving unit derives a combination of the number of scans and a percentage by which each scan overlaps an adjacent scan. The combination deriving unit derives the combination of the number of scans and the percentage such that the percentage is greater than a predetermined minimum value. The PET scanner according to the example further includes a setting unit configured to set a scan time for each of the scans based on the combination. The PET scanner according to the example further includes a scanning unit configured to scan the region of interest by performing each of the scans using the scan time. Each of these units is included in the CPU 170 described below using FIG. 10 and coordinates with a detector module etc.

A method of using a PET scanner of the example by imaging a region of interest in a subject, which is a region of interest having an axial extent greater than the length of an axial FOV of a PET scanner, includes: (1) a step of determining a combination of (a) a number that is the number of scans of overlapping scans and that is necessary to image the axial extent of the region of interest and (b) a percentage of the axial length of each scab by which each scan partly overlaps an adjacent scan, so that a total length of the determined number of overlapping scans successively performed exactly equals the axial extent of the region of interest; and (2) a step of setting a scan time for each of the scans, which are successively performed, based on the number and the overlap percentage that are determined at step (1). Each of the scans successively performed has the same axial length equal to the length of the axial FOV of the PET scanner.

Furthermore, as described below, a PET scanner according to an example includes a minimum number deriving unit, an excess length deriving unit, and a percentage deriving unit. The minimum number deriving unit derives a minimum number that is the number of scans in a case where multiple scans are successively performed while each scan overlaps an adjacent scan by an allowable minimum percentage and that is necessary to image at least an axial extent of a region of interest. The excess length deriving unit derives an excess length that is excessively scanned by the scans in the axial direction based on the minimum number of scans, the axial extent of the region of interest, and the length of the axial FOV. The percentage deriving unit derives a new percentage, which is different from the minimum percentage, as a percentage by which each scan overlaps an adjacent scan so that the excess length is zero. The PET scanner according to the example further includes a setting unit configured to set a scan time for each of the scans based on an efficiency gain E/L obtained from the new percentage, where E is the excess length and L is the axial extent of the region of interest. The PET scanner according to the example further includes a scanning unit configured to scan a subject by performing the minimum number of scans while each scan overlaps by the new percentage. Each of these units is included in the CPU 170 described below using FIG. 10 and coordinates with a detector module etc.

A method of using a PET scanner of the example by imaging a region of interest in a subject, which is a region of interest having an axial extent greater than the length of an axial FOV of a PET scanner, includes: (1) a step of determining a minimum number that is the number of scans of overlapping scans and that is necessary to image at least the axial extent of the region of interest; (2) a step of determining an excess length (total amount) that is excessively scanned by the scans in the axial direction based on the minimum number of scans determined in step (1), the axial extent of the region of interest, and the length of the axial FOV; and (3) a step of determining a new overlap percentage based on the excess length (total amount) and the minimum overlap percentage, so that a new total amount of the excess length (total amount) is zero. Each of the scans successively performed has the same axial length equal to the length of the axial FOV of the PET scanner. Each scan partly overlaps an adjacent scan by a predetermined percentage of the axial length of each scan.

Each embodiment will be described below.

In the first embodiment, no attempt is done to optimize the overlap, but the imaging resources are optimized on the area of interest. FIGS. 5 and 6 use the same example. The axial extent of the first scan shown in FIG. 6 is shifted from that shown in FIG. 5 so that the lower sensitivity part corresponds to a region outside the region of interest. Note that the "axial direction" means in general the longitudinal direction of a couchtop on which a subject is set or the direction of the body axis of the subject set on the couchtop.

In the first embodiment, the sum of wasted areas at the beginning and end of the scan (hereinafter, wasted area) has the same size as before, but there is lesser sensitivity in those areas. Thus, once the scan length between the ends is established and the number of steps is calculated to at least cover the entire area (and quite possibly more), the PET scanner splits the difference between the axial extent of the scan from end to end (overall axial extent) and the extent of the region of interest, at the beginning and end of the scan.

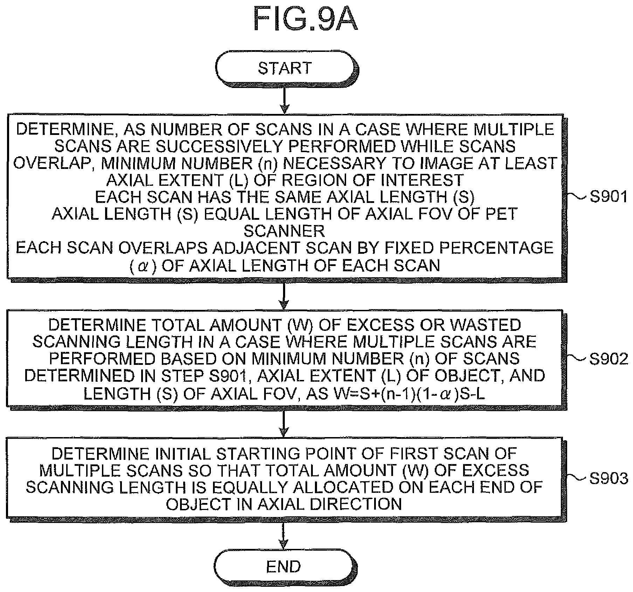

FIG. 9A illustrates the steps in a method of scanning according to the first embodiment. In particular, it is assumed that, at step S901, a fixed overlap in each step is $\alpha S (\alpha < 1)$. Here, S is the length of the axial FOV of the PET scanner and $\alpha$ is determined based on desired sensitivity (see below). The number of steps n in scanning is selected in step S901 so that the scanned distance D is greater than the axial extent L of the region of interest to be scanned, i.e., so that:

$$D = S + (n-1)(1-\alpha)S > L.$$

Further, in step S902, the wasted area (undershoot plus overshoot) is determined by the formula: W=D-L. In step S903, the starting point of the scan is selected so that the undershoot equals W/2 and the overshoot equals W/2.

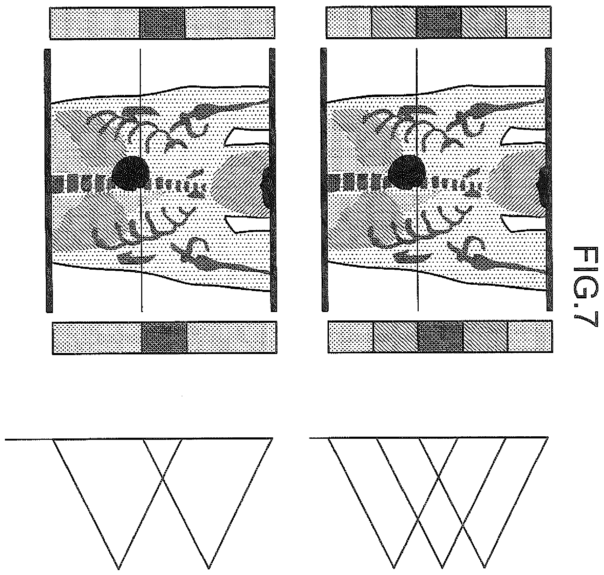

In a second example, the step size is adjusted to match the actual length of imaging. In addition, the scanning time is adjusted to compensate for the change in the step size. In other words, fewer, large steps would each be imaged for a relatively longer period of time, while more, smaller steps would each be imaged for a relatively shorter period of time. FIG. 7 illustrates an example of the shrinking of the step size (top view), an example of the stretching of the step size (bottom view), and the corresponding sensitivity profile. As is clear from FIG. 7, the adjustment of the step size will cause the overall axial sensitivity to no longer be flat.

It is supposed that the axial length is of exactly two steps with a 50% overlap. As the area to be covered is increased, the amount of overlap is reduced to match the increased distance. As the length of the scan increases, and based on either a threshold on axial sensitivity or on an arbitrary criterion set by the user, the overlap will be too small at some point. A third step is introduced with possible more that 50% overlap to match the distance.

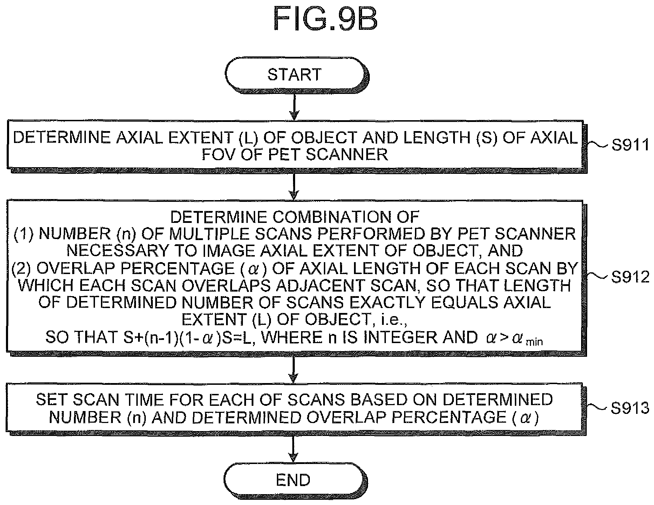

FIG. 9B illustrates the steps in a method of scanning according to the second example. In step S911, the axial extent to be scanned (L) and the length of the axial FOV (S) are determined.

In step S912, the combination of the percentage ($\alpha$) of the overlap in each step and the number (n) of steps in scanning are determined so that the scanned distance D is exactly equal to L, i.e., so that:

$$D = S + (n-1)(1-\alpha)S = L.$$

In the second example, $\alpha$ is constrained to be greater than a predetermined minimum value, and n is constrained to be an integer. In general, multiple solutions will exist in n-$\alpha$ space.

In the second example, the wasted area (undershoot plus overshoot), which is given by W=D-L, equals zero. Thus, in the second example, the starting point of the scan is selected as the beginning of the region of interest to be scanned.

In step S913, the scan time for each of the n scans is set based on the determined number n of scans and the determined overlap percentage $\alpha$. Typically, PET acquisition is defined to accumulate a certain total number of counts or a certain count density over the region of interest. The scanning time for a single step of a multi-step PET acquisition is selected to meet this requirement. If a new step size and/or a new overlap percentage are used, the overall efficiency of the utilization of imaging resources can be used to adjust the scanning time per step. For example, the two cases shown in FIG. 7 will be described. A desired nominal count density can be achieved with a scanning time of one minute per step. In the case of the top view exactly covering the desired length, three steps each require a scanning time of fifty seconds due to a larger overlap. In the case of the bottom view, two steps each require a scanning time of 75 seconds due to the smaller overlap. In general, the smaller the overlap, the longer the scanning time per step.

In a third example, the step sizes and the scanning method are a combination of the first and second which were set forth above. An algorithm is used to calculate the amount of overlap between steps and the amount of undershoot and overshoot along the axial direction of the image. First, for the nominal case, there is a fixed overlap $\alpha S(\alpha<1)$ in each step and a waste region $\beta S(\beta<1)$ at each end. They are expressed as a fraction S of the length of the axial FOV of the PET scanner. If L is the axial extent of the region of interest, which is to be scanned in n steps, the following relation holds:

$$L+2\beta S=S+(n-1)(1-\alpha)S \tag{1}$$

If L and S are given, n can be calculated along with $\alpha$ and $\beta$, subject to appropriate constraints. For example, with the constraints that $\beta$ is allowed to only decrease from a fixed initial value and, with respect to $\alpha$, $0.4<\alpha<0.6$ is allowed, this problem can be solved using a variety of optimization algorithms.

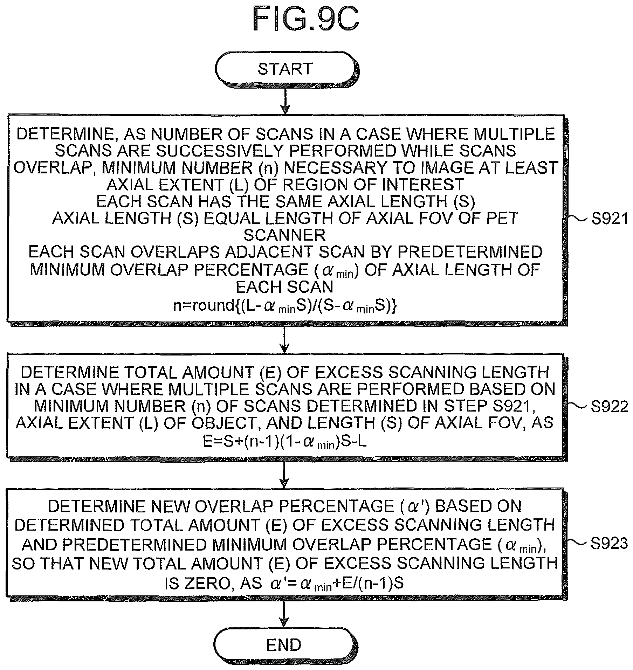

FIG. 9C illustrates steps in the method of scanning according to the third example.

Except for a waste region $\beta$ ($\beta=0$) and a minimum allowable value for $\alpha$ (i.e, $\alpha_{min}$), the optimization to find n and $\alpha$ is straightforward, as set forth.

If $\alpha$ is the minimum allowable value $\alpha_{min}$ of the overlap percentage ($\alpha=\alpha_{min}$) at step S921, then the number n of steps is derived from Equation (1), resulting in the equation:

$$n=round\{(L-\alpha_{min}S)/(S-\alpha_{min}S)\}, \tag{2}$$

with the division using a round-up function to rounded up to the next integer.

In step S922, E that is the extra length (hereinafter, excess length) in a case where n steps are performed with the percentage of the minimum allowable overlap is calculated as:

$$E(\alpha_{min})=S+(n-1)(1-\alpha_{min})S-L, \tag{3}$$

which is again based on Equation (1).

In step S923, letting $\alpha'=\alpha_{min}+\Delta\alpha$, setting $E(\alpha')=0$ in Equation (3), and solving for $\Delta\alpha$ results in an optimal overlap $\alpha'$ for which there is no extra space:

$$\alpha'=\alpha_{min}+E(\alpha_{min})/(n-1)S \tag{4}$$

For example, when a 55 cm region of interest is examined using a PET scanner having a 20 cm length of the axial FOV with a minimum overlap percentage of 50%, the number of steps according to Equation 2 is:

$$n=round\{(55-(0.5)(20))/(20-(0.5)(20))\}=5,$$

but the extra distance E by n=5 steps, according to Equation 3, is:

$$E=20+(5-1)(1-0.5)(20)-55=5 \text{ cm}.$$

The percentage of new overlap to produce exactly a 55 cm scan is as follows according to Equation 4:

$$\alpha'=0.5+5/(5-1)(20)=0.5625 \text{ or } 56.25\%.$$

Thus, approximately 9% (5/55) of the imaging resources is used to directly support the requested region of interest. The approximately 9% of the imaging resources can either be used to contribute to improve the image quality (9% more counts over the region of interest) or be used to reduce the scanning time per step by 9% to the preference of the user.

The same case with a 32 cm scanner would result in n=3, E=9 cm, $\alpha$'=64% and an overall better imaging resource utilization of 16%.

Similarly, additional constraints can be set for the beginning and end wasted areas. In practice, the user gives overall imaging preferences, such as acceptable variation in axial uniformity and minimum slice sensitivity. In some cases, depending on the set preferences, multiple combinations of steps and overlap percentage could exist.

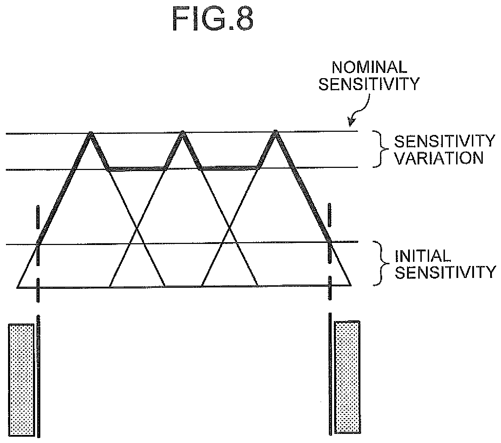

In reference to FIG. 8, an overall sensitivity profile is shown (bold line). Any given point of the sensitivity profile is composed from the summation of all data from all scanning steps. In a full 3D acquisition mode, a ring of the length S of the FOV will produce a triangular sensitivity profile with a peak at S/2. Depending on the overlap percentage $\alpha$, the overall sensitivity at any given scanning position will be the sum of one or more of the triangular sensitivity profiles. In other words, $C(x)=T(x)+T(x-S+\alpha S)+...$, where $T(x)$ is a first triangle, $T(x-S+\alpha S)$ is a second (shifted) triangle partly overlapping the first triangle by only $\alpha S$.

As described above, the feature that is targeted in the imaging protocol is the total number of counts (or count density). Accordingly, the clinical user would specify the minimum initial sensitivity (at the beginning and end of the scan) and the maximum (nominal) variation (in the central part of the scan). While the user is interested more in the sensitivity along the vertical axis, the system needs to convert the user's sensitivity requirements into movement along the horizontal axis. Clearly, the minimum initial sensitivity is directly proportional to the $\beta$ introduced earlier. In other words, $\beta=0.5*$ (initial sensitivity/nominal sensitivity). Similarly, the overlap percentage $\alpha$ directly affects the nominal sensitivity. In the second example described above, the overlap percentage $\alpha$ can be determined by determining the overlap percentage $\alpha$ that results in the function $C(x)$ having a maximum value equal to the nominal sensitivity set by the user.

In the previous example of L=55 cm and the length of the FOV of the PET scanner of S=32 cm, one result is steps of n=3 with an overlap percentage of $\alpha$'=64%. However, other solutions are steps of n=2 with an overlap percentage of $\alpha$'=22% and steps of n=4 with an overlap percentage of $\alpha$'=76%. Usually, the smaller number of steps is preferable, and a constraint on uniformity will establish the best option. For example, having only an overlap percentage of $\alpha$'=22% would typically result in an unacceptable reduction in sensitivity in the middle of the PET scanner. Depending of the overall allowable parameters, more complex optimization algorithms can be used.

The various methods set forth above will optimize the use of valuable imaging resources and provide the best possible information on the selected region of interest. The conventional technology of fixed step-and-shoot will be at more and more of a disadvantage as PET scanners with a greater length of the axial FOV are produced, and as more and more applications require fewer steps.

Thus, the various embodiments described above will provide an optimal utilization of valuable imaging resources by minimizing the waste area outside the requested imaging range and taking into account image quality and scanner sensitivity limits.

FIG. 10 is a schematic drawing of a gamma ray detection system that can be used to obtain a gamma ray or PET event information according to the embodiments. The PET scanner in each of the above-described embodiments includes, for example, the gamma ray detection system shown in FIG. 10. As shown in FIG. 10, a PMT (photomultiplier tube) 135 and a PMT 140 are arranged over a light guide 130 and the array of scintillation crystals 105 is arranged beneath the light guide 130. A second array of scintillation crystals 125 is disposed opposite the scintillation crystals 105 with a light guide 115, a photomultiplier tube 195, and a photomultiplier tube 110 arranged thereover. The photomultiplier tubes, the light guide, and the scintillation crystals can form a detector module, wherein the gamma ray detection system includes a plurality of detector modules arranged in a ring.

When gamma rays are emitted from a subject under test (not shown) in the gamma ray detection system shown in FIG. 10, the two gamma rays are emitted in opposite directions, approximately 180° from each other. When the gamma rays are detected at the scintillation crystal 105 and the scintillation crystal 125 within a predefined time limit, a scintillation event is determined. Thus, the gamma ray detection system detects gamma rays simultaneously at the scintillation crystal 105 and the scintillation crystal 125. For simplicity only, gamma ray detection is described relative to the scintillation crystal 105. One of ordinary skill in the art will recognize, however, that the description given herein with respect to the scintillation crystal 105 is equally applicable to gamma ray detection at the scintillation crystal 125.

The photomultiplier tube 110, the photomultiplier tube 135, the photomultiplier tube 140, and the photomultiplier tube 195 are each connected to a data acquisition device 150. The data acquisition device 150 includes hardware configured to process the signals from the photomultiplier tubes. The data acquisition device 150 measures the arrival time of the gamma ray. The data acquisition device 150 produces two outputs (one for the combination of the photomultiplier tube 135 and the photomultiplier tube 140 and one for the combination of the photomultiplier tube 110 and the photomultiplier tube 195) which encodes the time of the discriminator pulse relative to a system clock (not shown). For a TOF PET scanner, the data acquisition device 150 typically produces a time stamp with an accuracy of 15 to 25 picoseconds. The data acquisition device 150 measures the amplitude of the signal on each photomultiplier tube (four outputs from the data acquisition device 150).

The outputs from the data acquisition device 150 are provided to the CPU 170 and processed. The processing steps consist of steps of estimating an energy and a detection position from the outputs of the data acquisition device 150 and an arrival time from the time stamp outputs for each event. The CPU 170 may include the application of many correction steps to improve the accuracy of the energy, the detection position, and estimates of the arrival time. As one of ordinary skill in the art would recognize, the CPU 170 can be implemented as discrete logic gates, using an ASIC (Application Specific Integrated Circuit), a FPGA (Field Programmable Gate Array) or other CPLD (Complex Programmable Logic Device). An FPGA or CPLD implementation may be coded in VHDL (Very High speed integrated circuit hardware Description Language), Verilog or any other hardware description language. The code may be stored in an electronic memory directly within the FPGA or CPLD. Alternatively, the code may be stored as a separate electronic memory. Further, the electronic memory may be nonvolatile, such as a ROM (Read Only Memory), a EPROM (Erasable Programmable Read Only Memory), a EEPROM (Electrically Erasable Programmable Read Only Memory) or a FLASH memory. The electronic memory may also be volatile, such as a static RAM (Raddom Access Memory) or a dynamic RAM. A processor, such as a microcontroller or a microprocessor, may be provided to manage the electronic memory as well as the interaction between the FPGA or the CPLD and the electronic memory.

Alternatively, the CPU 170 may execute a computer program including a set of computer-readable instructions to perform the functions described herein. The program is stored in any of the above-described non-transitory electronic memories and/or a hard disk device, a CD (Compact Disc), a DVD (Digital Versatile Disc), a FLASH drive or any other known storage media. Further, the computer-readable instructions may be provided as a utility application, a background daemon, or a component of an operating system, or a combination thereof, executing in conjunction with a processor, such as a Xenon processor (trademark) from Intel of America or an Opteron processor (trademark) from AMD (Advanced Micro Devices, Inc.) of America, and an operating system, such as Microsoft VISTA (trademark), UNIX (trademark), Solaris (trademark), LINUX (trademark), Apple (trademark), MAC-OS (trademark), and other operating systems known to those skilled in the art.

Once processed by the CPU 170, the CPU 170 stores the processed signals in an electronic storage 180 and/or displays the processed signals on a display 145. As one of ordinary skill in the art would recognize, the electronic storage 180 may be a hard disk, a CD-ROM, a DVD, a FLASH, a RAM, a ROM or any other electronic storage known in the art. The display 145 may be implemented as an LCD display (Liquid Crystal Display), a CRT display (Cathode Ray Tube), a plasma display, an OLED (Organic Light Emitting Diode), a LED (Light Emitting Diode) or any other display known in the art. As such, the descriptions of the electronic storage 180 and the display 145 provided herein are merely exemplary and in no way limit the scope of the present advancements.

The gamma ray detection system shown in FIG. 10 also includes an interface 175 that interfaces with other external devices and/or a user. For example, the interface 175 may be a USB (Universal Serial Bus) interface, a PCMCIA (Personal Computer Memory Card International Association) interface, an Ethernet (trademark) interface or any other interface known in the art. The interface 175 may also be wired or wireless and may include a keyboard and/or a mouse or other human interface devices known in the art for interacting with a user.

The positron emission computed tomography apparatus of at least one of the above-described embodiments, the method that is performed by a positron emission computed tomography apparatus, and the program allows efficient imaging.

**Claims**

1.   A positron emission computed tomography apparatus comprising:

a minimum number deriving unit (170) adapted to derive a minimum number that is the number of scans in a case where multiple scans are successively performed while each scan overlaps an adjacent scan by a predetermined percentage and that is necessary to image at least an axial extent of a region of interest,
an excess length deriving unit (170) adapted to derive an excess length that is excessively scanned by the scans in the axial direction based on the minimum number of scans, the axial extent of the region of interest, and the length of an axial FOV, Field Of View;
a starting point deriving unit (170) adapted to derive a starting point of a first scan of the scans so that the excess length is equally allocated on each end of the region of interest in the axial direction; and
a scanning unit (170) adapted to scan a subject by starting the minimum number of scans from the starting point.

2.   A method performed by a positron emission computed tomography apparatus, the method comprising steps of:

deriving a minimum number that is the number of scans in a case where multiple scans are successively performed while each scan overlaps an adjacent scan by a predetermined percentage and that is necessary to image at least an axial extent of a region of interest,
deriving an excess length that is excessively scanned by the scans in the axial direction based on the minimum number of scans, the axial extent of the region of interest, and the length of an axial FOV, Field Of View;
deriving a starting point of a first scan of the scans so that the excess length is equally allocated on each end of the region of interest in the axial direction; and
scanning a subject by starting the minimum number of scans from the starting point.

3. A program that causes a position emission computed tomography apparatus to execute procedures of:

deriving a minimum number that is the number of scans in a case where multiple scans are successively performed while each scan overlaps an adjacent scan by a predetermined percentage and that is necessary to image at least an axial extent of a region of interest,
deriving an excess length that is excessively scanned by the scans in the axial direction based on the minimum number of scans, the axial extent of the region of interest, and the length of an axial FOV, Field Of View;
deriving a starting point of a first scan of the scans so that the excess length is equally allocated on each end of the region of interest in the axial direction; and
scanning a subject by starting the minimum number of scans from the starting point.

## Patentansprüche

1. Positronen-Emissions-Computertomographievorrichtung, Folgendes umfassend:

eine Minimalanzahl-Ableitungseinheit (170), dazu angepasst, eine Minimalanzahl abzuleiten, das heißt die Anzahl von Abtastungen in einem Fall ist, wo mehrfache Abtastungen nacheinander ausgeführt werden, während jede Abtastung eine benachbarte Abtastung um einen vorgegebenen Prozentsatz überlappt, und was erforderlich ist, um mindestens eine axiale Ausdehnung eines Bereichs von Interesse abzubilden,
eine Überlängen-Ableitungseinheit (170), dazu angepasst, eine Überlänge abzuleiten, die durch die Abtastungen in der axialen Richtung übermäßig abgetastet wird, auf der minimalen Anzahl von Abtastungen, der axialen Ausdehnung des Bereichs von Interesse und der Länge eines Gesichtsfelds, FoV, basierend;
eine Startpunkt-Ableitungseinheit (170), dazu angepasst, einen Startpunkt einer ersten Abtastung der Abtastungen abzuleiten, so dass die Überlänge auf jedem Ende des Bereichs von Interesse in der axialen Richtung gleich zugewiesen wird; und
eine Abtasteinheit (170), dazu angepasst, ein Objekt abzutasten, indem die minimale Anzahl von Abtastungen vom Startpunkt gestartet wird.

2. Verfahren, ausgeführt von einer Positronen-Emissions-Computertomographievorrichtung, wobei das Verfahren die folgenden Schritte umfasst:

Ableiten einer minimalen Anzahl, was die Anzahl von Abtastungen in einem Fall ist, wo mehrfache Abtastungen nacheinander ausgeführt werden, während jede Abtastung eine benachbarte Abtastung um einen vorgegebenen Prozentsatz überlappt und was erforderlich ist, um mindestens eine axiale Ausdehnung eines Bereichs von Interesse abzubilden;
Ableiten einer Überlänge, die übermäßig abgetastet wird durch die Abtastungen in der axialen Richtung auf der Basis der minimalen Anzahl von Abtastungen, der axialen Ausdehnung des Bereichs von Interesse und der Länge eines axialen Gesichtsfelds, FoV;
Ableiten eines Startpunkts einer ersten Abtastung der Abtastungen, sodass die Überlänge auf jedem Ende des Bereichs von Interesse in der axialen Richtung gleich zugewiesen wird; und
Abtasten eines Objekts durch Starten der minimalen Anzahl von Abtastungen vom Startpunkt.

3. Programm, das eine Positronen-Emissions-Computertomographievorrichtung veranlasst, folgende Prozeduren auszuführen:

Ableiten einer minimalen Anzahl, was die Anzahl von Abtastungen in einem Fall ist, wo mehrfache Abtastungen nacheinander ausgeführt werden, während jede Abtastung eine benachbarte Abtastung um einen vorgegebenen Prozentsatz überlappt, und was erforderlich ist, um mindestens eine axiale Ausdehnung eines Bereichs von Interesse abzubilden;
Ableiten einer Überlänge, die von den Abtastungen in der axialen Richtung übermäßig abgetastet wird, auf der minimalen Anzahl von Abtastungen, der axialen Ausdehnung des Bereichs von Interesse und der Länge eines axialen Gesichtsfelds, FoV, basierend;

Ableiten eines Startpunkts einer ersten Abtastung der Abtastungen, sodass die Überlänge auf jedem Ende des Bereichs von Interesse in der axialen Richtung gleich zugewiesen wird; und

Abtasten eines Objekts durch Starten der minimalen Anzahl von Abtastungen vom Startpunkt.

## Revendications

1. Appareil de tomographie assistée par ordinateur à émission de positons comprenant :

   une unité de dérivation de nombre minimum (170) qui est adaptée de manière à dériver un nombre minimum qui est le nombre de balayages dans un cas où de multiples balayages sont réalisés en succession tandis que chaque balayage chevauche un balayage adjacent selon un pourcentage prédéterminé et qui est nécessaire pour imager au moins une étendue axiale d'une région d'intérêt ;
   une unité de dérivation de longueur en excès (170) qui est adaptée de manière à dériver une longueur en excès qui est balayée en excès par les balayages dans la direction axiale sur la base du nombre minimum de balayages, de l'étendue axiale de la région d'intérêt et de la longueur d'un champ de vision, FOV, axial ;
   une unité de dérivation de point de début (170) qui est adaptée de manière à dériver un point de début d'un premier balayage des balayages de telle sorte que la longueur en excès soit répartie de façon égale sur chaque extrémité de la région d'intérêt dans la direction axiale ; et
   une unité de balayage (170) qui est adaptée de manière à balayer un sujet en débutant le nombre minimum de balayages depuis le point de début.

2. Procédé réalisé par un appareil de tomographie assistée par ordinateur à émission de positons, le procédé comprenant les étapes constituées par :

   la dérivation d'un nombre minimum qui est le nombre de balayages dans un cas où de multiples balayages sont réalisés en succession tandis que chaque balayage chevauche un balayage adjacent selon un pourcentage prédéterminé et qui est nécessaire pour imager au moins une étendue axiale d'une région d'intérêt ;
   la dérivation d'une longueur en excès qui est balayée en excès par les balayages dans la direction axiale sur la base du nombre minimum de balayages, de l'étendue axiale de la région d'intérêt et de la longueur d'un champ de vision, FOV, axial ;
   la dérivation d'un point de début d'un premier balayage des balayages de telle sorte que la longueur en excès soit répartie de façon égale sur chaque extrémité de la région d'intérêt dans la direction axiale ; et
   le balayage d'un sujet en débutant le nombre minimum de balayages depuis le point de début.

3. Programme qui force un appareil de tomographie assistée par ordinateur à émission de positons à exécuter des procédures constituées par :

   la dérivation d'un nombre minimum qui est le nombre de balayages dans un cas où de multiples balayages sont réalisés en succession tandis que chaque balayage chevauche un balayage adjacent selon un pourcentage prédéterminé et qui est nécessaire pour imager au moins une étendue axiale d'une région d'intérêt ;
   la dérivation d'une longueur en excès qui est balayée en excès par les balayages dans la direction axiale sur la base du nombre minimum de balayages, de l'étendue axiale de la région d'intérêt et de la longueur d'un champ de vision, FOV, axial ;
   la dérivation d'un point de début d'un premier balayage des balayages de telle sorte que la longueur en excès soit répartie de façon égale sur chaque extrémité de la région d'intérêt dans la direction axiale ; et
   le balayage d'un sujet en débutant le nombre minimum de balayages depuis le point de début.

# FIG.1

# FIG.2

TYPICAL WHOLE BODY
~200 M COUNTS

# FIG.3

TYPICAL WHOLE BODY
~200 M COUNTS

# FIG.4

# FIG.5

# FIG.6

# FIG.7

# FIG.8

# FIG.9A

```
START
```

DETERMINE, AS NUMBER OF SCANS IN A CASE WHERE MULTIPLE SCANS ARE SUCCESSIVELY PERFORMED WHILE SCANS OVERLAP, MINIMUM NUMBER (n) NECESSARY TO IMAGE AT LEAST AXIAL EXTENT (L) OF REGION OF INTEREST EACH SCAN HAS THE SAME AXIAL LENGTH (S) AXIAL LENGTH (S) EQUAL LENGTH OF AXIAL FOV OF PET SCANNER EACH SCAN OVERLAPS ADJACENT SCAN BY FIXED PERCENTAGE ($\alpha$) OF AXIAL LENGTH OF EACH SCAN — S901

DETERMINE TOTAL AMOUNT (W) OF EXCESS OR WASTED SCANNING LENGTH IN A CASE WHERE MULTIPLE SCANS ARE PERFORMED BASED ON MINIMUM NUMBER (n) OF SCANS DETERMINED IN STEP S901, AXIAL EXTENT (L) OF OBJECT, AND LENGTH (S) OF AXIAL FOV, AS $W=S+(n-1)(1-\alpha)S-L$ — S902

DETERMINE INITIAL STARTING POINT OF FIRST SCAN OF MULTIPLE SCANS SO THAT TOTAL AMOUNT (W) OF EXCESS SCANNING LENGTH IS EQUALLY ALLOCATED ON EACH END OF OBJECT IN AXIAL DIRECTION — S903

```
END
```

# FIG.9B

START

DETERMINE AXIAL EXTENT (L) OF OBJECT AND LENGTH (S) OF AXIAL FOV OF PET SCANNER ∼S911

DETERMINE COMBINATION OF
(1) NUMBER (n) OF MULTIPLE SCANS PERFORMED BY PET SCANNER NECESSARY TO IMAGE AXIAL EXTENT OF OBJECT, AND
(2) OVERLAP PERCENTAGE ($\alpha$) OF AXIAL LENGTH OF EACH SCAN BY WHICH EACH SCAN OVERLAPS ADJACENT SCAN, SO THAT LENGTH OF DETERMINED NUMBER OF SCANS EXACTLY EQUALS AXIAL EXTENT (L) OF OBJECT, i.e.,
SO THAT S+(n-1)(1-$\alpha$)S=L, WHERE n IS INTEGER AND $\alpha > \alpha_{min}$ ∼S912

SET SCAN TIME FOR EACH OF SCANS BASED ON DETERMINED NUMBER (n) AND DETERMINED OVERLAP PERCENTAGE ($\alpha$) ∼S913

END

# FIG.9C

START

DETERMINE, AS NUMBER OF SCANS IN A CASE WHERE MULTIPLE
SCANS ARE SUCCESSIVELY PERFORMED WHILE SCANS
OVERLAP, MINIMUM NUMBER (n) NECESSARY TO IMAGE AT LEAST
AXIAL EXTENT (L) OF REGION OF INTEREST
EACH SCAN HAS THE SAME AXIAL LENGTH (S)
AXIAL LENGTH (S) EQUAL LENGTH OF AXIAL FOV OF PET
SCANNER
EACH SCAN OVERLAPS ADJACENT SCAN BY PREDETERMINED
MINIMUM OVERLAP PERCENTAGE ($\alpha_{min}$) OF AXIAL LENGTH OF
EACH SCAN
$n = round\{(L - \alpha_{min}S)/(S - \alpha_{min}S)\}$

~ S921

DETERMINE TOTAL AMOUNT (E) OF EXCESS SCANNING LENGTH
IN A CASE WHERE MULTIPLE SCANS ARE PERFORMED BASED ON
MINIMUM NUMBER (n) OF SCANS DETERMINED IN STEP S921,
AXIAL EXTENT (L) OF OBJECT, AND LENGTH (S) OF AXIAL FOV, AS
$E = S + (n-1)(1 - \alpha_{min})S - L$

~ S922

DETERMINE NEW OVERLAP PERCENTAGE ($\alpha'$) BASED ON
DETERMINED TOTAL AMOUNT (E) OF EXCESS SCANNING LENGTH
AND PREDETERMINED MINIMUM OVERLAP PERCENTAGE ($\alpha_{min}$),
SO THAT NEW TOTAL AMOUNT (E) OF EXCESS SCANNING LENGTH
IS ZERO, AS $\alpha' = \alpha_{min} + E/(n-1)S$

~ S923

END

# FIG.10

**EP 2 667 222 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- Database. 11779807